(19) **Europäisches Patentamt / European Patent Office / Office européen des brevets**

(11) **EP 2 752 186 B1**

(12) **EUROPEAN PATENT SPECIFICATION**

(45) Date of publication and mention of the grant of the patent:
**02.11.2016 Bulletin 2016/44**

(21) Application number: **12828224.1**

(22) Date of filing: **30.08.2012**

(51) Int Cl.:
*A61K 8/39* (2006.01)  *A61K 8/46* (2006.01)
*A61Q 19/10* (2006.01)  *C11D 1/06* (2006.01)
*C11D 1/14* (2006.01)  *C11D 1/29* (2006.01)
*C11D 1/66* (2006.01)

(86) International application number:
**PCT/JP2012/072018**

(87) International publication number:
**WO 2013/031899 (07.03.2013 Gazette 2013/10)**

(54) **SKIN CLEANSER COMPOSITION**

HAUTREINIGUNGSZUSAMMENSETZUNG

COMPOSITION NETTOYANTE POUR LA PEAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2011 JP 2011191786**

(43) Date of publication of application:
**09.07.2014 Bulletin 2014/28**

(73) Proprietor: **Kao Corporation**
**Chuo-Ku**
**Tokyo 103-8210 (JP)**

(72) Inventors:
• **MASUI, Takashi**
**Tokyo 131-8501 (JP)**

• **TAKEUCHI, Hiroki**
**Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**EP-A1- 0 417 501      EP-A1- 2 612 652**
**EP-A2- 1 982 692      WO-A1-2012/029950**
**JP-A- S6 121 199      JP-A- H02 175 799**
**JP-A- H03 103 500      JP-A- H03 103 500**
**JP-A- H08 283 974      JP-A- H10 110 187**
**JP-A- 2006 282 662      JP-A- 2008 285 479**
**US-A1- 2006 233 733**

**Description**

[Field of the Invention]

The present invention relates to a skin cleansing composition.

[Background of the Invention]

In the regions of Asia including Japan, soap has been traditionally used as a primary skin cleanser used for, for example, washing the whole body. Soap has been favored by consumers in these regions for its favorable lathering performance and creamy foam qualities as well as a feeling of friction during rinsing. However, soap had a problem with, for example, mildness to the skin in such a way that soap made skin feel tight (Non Patent Publication 1).

On the other hand, alkyl ether sulfate has been used as a skin cleanser used for, for example, washing the whole body, mainly in the U.S. and Europe. However, alkyl ether sulfate has bubbly foam qualities and gives a slimy feel during rinsing; therefore, it is not popularly accepted in Japan and other regions of Asia (Patent Publication 1). Further, although alkyl ether sulfate is relatively milder to the skin than soap, its mildness is still unsatisfactory.

Moreover, although alkyl ether carboxylic acid-based surfactants are known to be less skin-irritating surfactants, they had problems of, for example, poor lathering performance and a slimy feel during rinsing.

In order to solve these problems, a cleansing composition containing an ether carboxylic acid-based surfactant having a specific distribution of moles of ethylene oxide added (Patent Publication 2), a cleansing composition containing a combination of an alkyl ether carboxylic acid-based surfactant and a cationic polymer (Patent Publication 3), and the like have been studied. However, each of these cleansing compositions also has problems of, for example, insufficient effect and poor versatility due to a complex system.

Further, the combined use of alkyl ether carboxylic acid-based surfactants with other surfactants such as alkyl ether sulfate has also been studied (Patent Publication 4); however, effects in terms of lathering performance and rinsing are still insufficient. Moreover, there have been problems, for example, that the foam obtained with these cleansing compositions was watery and when these cleansing compositions were used for washing large areas such as the whole body, the foam poorly spread out.

[Citation List]

[Non Patent Publication]

[Non Patent Publication 1] Surfactants in Cosmetics, Second Edition, P. 427

[Patent Publication]

[Patent Publication 1] International Publication No. WO96/0579
[Patent Publication 2] JP-A-H02-175799
[Patent Publication 3] JP-A-2009-263290
[Patent Publication 4] JP-A-2008-285479

[Summary of the Invention]

The present invention provides a skin cleansing composition, comprising the following components (A) and (B) :

(A) an alkyl ether carboxylic acid or a salt thereof represented by formula (1):

$$R^1\text{-O-}(CH_2CH_2O)_n\text{-}CH_2\text{-COOM} \qquad (1)$$

wherein, $R^1$ represents an alkyl group having 10 to 16 carbon atoms, n represents a number of from 0 to 20, and M represents a hydrogen atom, alkali metal, alkaline earth metal, ammonium, or organic ammonium,
wherein, $R^1$ has an average carbon number of from 10.8 to 12.8,
and wherein, the alkyl ether carboxylic acid or a salt thereof contains a component in which n = 0 in an amount of 9.8% to 27% by mass, and the total content of a component in which n = 1 and a component in which n = 2 is 21% by mass or more and less than 40% by mass, and an average value of n is from 2.8 to 3.4, and

(B) a polyoxyethylene alkyl sulfate represented by formula (2):

$$R^2\text{-O-}(CH_2CH_2O)_m\text{-}SO_3Y \qquad (2)$$

wherein, $R^2$ represents an alkyl group or an alkenyl group having 8 to 22 carbon atoms, m represents a number of from 0 to 20 and an average of m is 2 or more and less than 4, and Y represents a hydrogen atom or a cation selected from alkali metal, alkaline earth metal, ammonium, alkyl ammonium, alkanol ammonium, and glucammonium.

[Detailed Description of the Invention]

The present invention relates to a skin cleansing composition which is mild to the skin and has excellent lathering performance, volume of foam, foam qualities, and rinsing properties. Moreover, the foam produced with the skin cleansing composition does not easily drip off the skin but is easily spread on the skin.

The present inventors found that a skin cleansing composition which is mild to the skin and has excellent lathering performance, volume of foam, foam qualities, and rinsing properties, in which the foam produced does not easily drip off the skin but is easily spread on the skin, can be obtained by using an alkyl ether carboxylate having a specific distribution in combination with a specific alkyl sulfate or polyoxyethylene alkyl sulfate.

The skin cleansing composition of the present invention is mild to the skin and has excellent lathering performance, volume of foam and foam qualities, and in addition, favorable rinsing properties. Moreover, the foam produced with the skin cleansing composition of the present invention does not easily drip off the skin but is easily spread on the skin, and thus is suitable for washing the skin.

The alkyl ether carboxylic acid or a salt thereof of the component (A) used in the present invention is represented by formula (1).

In the formula, $R^1$ is an alkyl group having 10 to 16 carbon atoms. Also, although the alkyl chain of $R^1$ may be either linear or branched, from the viewpoint of foaming properties, a linear alkyl group is preferred. Also, $R^1$ has an average carbon number of from 10.8 to 12.8, preferably from 10.8 to 12.5, and more preferably from 12.1 to 12.4. It is preferable that the average carbon number be within the above range since excellent foaming properties, foam qualities, and stability at low temperature are obtained.

Also, $R^1$ preferably contains two or more alkyl groups, and the content of a component having the alkyl chain length which is contained in the highest content is preferably 55% by mass or more and less than 97% by mass, more preferably from 60 to 95% by mass, and even more preferably from 70 to 95% by mass since excellent volume of foam and foam qualities are obtained.

Further, in the formula, n represents a number of from 0 to 20, preferably from 0 to 12. Also, n represents the number of moles of ethylene oxide added, and an average number of moles added (an average value of n) in the composition of the component (A) is from 2.8 to 3.4, and further preferably from 2.8 to 3.1 since favorable lathering performance is achieved.

The alkyl ether carboxylic acid or a salt thereof of the component (A) contains, in formula (1), a component in which n = 0 in an amount of from 9.8 to 27% by mass, more preferably from 9.9 to 27% by mass, even more preferably from 9.9 to 16% by mass, and even more preferably from 9.9 to 15% by mass. When the content of the component in which n = 0 is within the above range, excellent volume of foam and foam qualities are achieved.

Further, preferably, the component (A) contains a component in which n = 1 and a component in which n = 2 in a total amount of 21% by mass or more and less than 40% by mass, preferably from 27 to 37% by mass, more preferably from 27 to 36.5% by mass, and even more preferably from 35 to 36.1% by mass from the viewpoint of foam qualities and the volume of foam.

Also, in the formula, examples of M include a hydrogen atom; alkali metal such as sodium and potassium; alkaline earth metal such as calcium and magnesium; ammonium; alkanolamine-derived ammonium such as monoethanolamine, diethanolamine, and triethanolamine. Among them, alkali metal is preferred in terms of foaming properties, stability at low temperature, and absence of coloration over time.

In formula (1), the alkyl ether carboxylic acid or a salt thereof of the component (A) preferably has a mass ratio of the components in which n = 0, 1, 2, 3, and 4, (the mass of a component in which n = 0) : (the mass of a component in which n = 1) : (the mass of a component in which n = 2) : (the mass of a component in which n = 3) : (the mass of a component in which n = 4), of 1 : 0.99 to 3.50 : 0.89 to 3.00 : 0.76 to 3.00 : 0.63 to 1.52 from the viewpoint that foaming properties, detergency, and a feeling of friction during rinsing can be achieved simultaneously.

Also, in formula (1), it is preferable that the content of a component in which n = 0 be 9.9% by mass or more and less than 12% by mass, and a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52, or the content of a component in which n = 0 be 12% by mass or more and 17% by mass or less and a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99 from the viewpoint of foam qualities and volume of foam.

Further, in formula (1), it is preferable that the content of a component in which n = 0 be from 9.9 to 11.5% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.58 to 1.84 : 1.72 to 2.17 : 1.49 to 2.00 : 1.14 to 1.52 or, in formula (1), it is preferable that the content of a component in which n = 0 be from 13 to 15% by mass and a ratio of (the mass of a component in which n = 0) : (the mass of a component in which n = 1) : (the mass of a component in which n = 2) : (the mass of a component in which n = 3) : (the mass of a component in which n = 4) = 1 : 1.00 to 1.31 : 0.93 to 1.34 : 0.79 to 1.18 : 0.63 to 0.99 from the viewpoint of foam qualities and volume of foam.

It is preferable that, in the component (A), in formula (1), $R^1$ be an alkyl group having 10 to 16 carbon atoms, $R^1$ have an average carbon number of from 12.1 to 12.4, and the content of a component having the alkyl chain length which is contained in the highest content be from 70 to 95% by mass, and further, n represent a number of from 0 to 20 and the average value of n be from 2.8 to 3.1, and a component in which n = 0 be contained in an amount of from 9.9 to 15% by mass, and a component in which n = 1 and a component in which n = 2 be contained in a total amount of from 35 to 36.1% by mass. Also, as M in the formula, a hydrogen atom, sodium, potassium, and ammonium are preferred. A cleansing composition containing the alkyl ether carboxylic acid or a salt thereof having the aforementioned configuration can have improved volume of foam and foam qualities.

It is to be noted that in the component (A) of the present invention, the distribution of the alkyl chain length of $R^1$, the average alkyl chain length of $R^1$, the amount of a component in which n = 0, the average number of added moles n, and a mass ratio of the components in which n = 0, 1, 2, 3, and 4 are obtained as follows from the gas chromatographic analysis of the alkyl ether carboxylic acid represented by formula (1).

[Distribution of the alkyl chain length of $R^1$]

From the peak areas obtained by gas chromatography, a peak area of each alkyl chain length corresponding to n = 0 mole was obtained, and setting the sum of the peak areas thus obtained at 100, the percentage of the distribution of each alkyl chain length was calculated. Similar calculation was carried out also as to n = 1 to 3 moles, and the percentage values of the distribution of each alkyl chain length corresponding to n = 0 to 3 moles were averaged out, whereby the distribution of the alkyl chain length of $R^1$ was obtained (from this, the alkyl group component contained in the largest amount in the composition of $R^1$ can be specified).

[Average alkyl chain length of $R^1$]

From the distribution of the alkyl chain length of $R^1$ obtained as above, the proportion of each component was obtained, which was multiplied by the number of carbon atoms of the corresponding alkyl chain length, and the resulting values were summed up. The value thus obtained was used as an average alkyl chain length.

[Amount of a component in which n = 0, total amount of a component in which n = 1 and a component in which n = 2]

In the composition of $R^1$, the alkyl chain length which is contained in the highest content was specified, and the peak areas of the component having alkyl chain length of the highest content corresponding to n = 0 to 10 were summed up by gas chromatography. By setting the total amount thus obtained at 100%, the amount of a component in which n = 0, the total amount of a component in which n = 1 and a component in which n = 2 were calculated.

[Average number of added moles n]

In the composition of $R^1$, the alkyl chain length of the highest content was specified, and the peak areas of the component having the alkyl chain length of the highest content corresponding to n = 0 to 10 were summed up by gas chromatography (the amount of a component in which n is 11 or more was so small that it was excluded from the calculation). By setting the total amount thus obtained at 1, each proportion of n = 0 to 10 was obtained. The resulting proportion was multiplied by each number of added moles, and the sum of the resulting values was used as the average number of added moles n.

[Mass ratio of the components in which n = 0, 1, 2, 3, and 4]

As to the mass ratio of each of the components having different numbers of moles of EO added, the distribution of the alkyl chain length of $R^1$ was obtained from the peak area obtained by gas chromatography by the method described above, and the component having the alkyl chain length of the highest content in the composition of $R^1$ was specified, and the ratio of each of the components having different numbers of moles of EO added was specified by the area ratio of n = 0, n = 1, n = 2, n = 3, and n = 4 of the component having the alkyl chain length of the highest content.

The alkyl ether carboxylic acid or a salt thereof of the component (A) has the aforementioned composition, and from the viewpoint of rinsing, the content thereof is preferably 0.1% by mass or more, more preferably 0.5% by mass or more, even more preferably 1% by mass or more and preferably 30% by mass or less, more preferably 20% by mass or less, and even more preferably 15% by mass or less of the total composition. Further, the content thereof is preferably from 0.1 to 30% by mass, more preferably from 0.5 to 20% by mass, and even more preferably from 1 to 15% by mass of the total composition.

The alkyl sulfate or polyoxyethylene alkyl sulfate of the component (B) used in the present invention is represented by the aforementioned formula (2).

In the formula, $R^2$ represents an alkyl group or an alkenyl group having 8 to 22 carbon atoms, which may be linear or branched. $R^2$ is preferably an alkyl group having 12 to 18 carbon atoms, and more preferably an alkyl group having 12 to 14 carbon atoms.

Further, in the formula, m represents a number of from 0 to 20, and more preferably from 0 to 12. The letter m represents a number of moles of ethylene oxide added, and the average number of moles added (an average value of m) of the component (B) in the composition is 2 or more and less than 4, and from the viewpoint of the volume of foam, the average value of m is preferably from 2 to 3.5.

Examples of Y include a hydrogen atom; alkali metal such as sodium and potassium; alkaline earth metal such as calcium and magnesium; ammonium, alkyl ammonium, alkanol ammonium, and glucammonium. Among them, alkali metal and ammonium are preferred in view of easy dissolution in water as well as high compatibility with water.

As the component (B), sodium polyoxyethylene (2) lauryl ether sulfate, sodium polyoxyethylene (3) lauryl ether sulfate, and the like are preferable.

Also, commercial products such as EMAL 227, EMAL 270, and EMAL 327 (all are manufactured by Kao Corporation) can be used.

The component (B) can be used alone or in combination of two or more thereof, and from the viewpoint of the volume of foam, the content thereof is preferably 0.1% by mass or more, more preferably 0.5% by mass or more, even more preferably 1% by mass or more and preferably 30% by mass or less, more preferably 20% by mass or less, and even more preferably 15% by mass or less of the total composition. Further, the content thereof is preferably from 0.1 to 30% by mass, more preferably from 0.5 to 20% by mass, and even more preferably from 1 to 15% by mass of the total composition.

When only the component (A) is added to the cleansing composition, the resulting foam is creamy and heavy, which cannot be easily spread by hands or a tool such as a towel. Meanwhile, when only the component (B) is added to the cleansing composition, the resulting foam is bubbly and watery, and when it is applied to the skin, it readily drips off. Further, the rinsing properties are deteriorated and the skin is likely to feel tight after washing.

According to the present invention, foam which is mild to the skin and has excellent lathering performance, volume of foam, and foam qualities as well as favorable rinsing properties, and which does not easily drip off the skin but is easily spread on the skin can be obtained by using a combination of these components (A) and (B).

The mass ratio of the component (A) to the component (B) in the cleansing composition of the present invention is preferably (A) : (B) = 1 : 10 to 10 : 1, more preferably (A) : (B) = 1 : 5 to 5 : 1, and even more preferably (A) : (B) = 1 : 2 to 2 : 1.

From the viewpoint of easy handling, the total content of the component (A) and the component (B) in the skin cleansing composition of the present invention is preferably 0.5% by mass or more, more preferably 1% by mass or more, and even more preferably 5% by mass or more and preferably 40% by mass or less, more preferably 30% by mass or less, and even more preferably 15% by mass or less of the total composition. Also, the content thereof is preferably from 0.5 to 40% by mass, more preferably from 1 to 30% by mass, and even more preferably from 5 to 15% by mass of the total composition.

The cleansing composition of the present invention can further comprise (C) a nonionic surfactant, thereby further improving the volume of foam and foam qualities without affecting mildness to the skin.

Examples of the component (C) include glycerin fatty acid ester, propylene glycol fatty acid ester, sorbitan fatty acid ester, polyoxyethylene sorbitan fatty acid ester, polyoxyethylene alkyl ether, polyoxyethylene polyoxypropylene glycol, alkyl glyceryl ether, polyethylene glycol fatty acid ester, alkyl alkanolamide, alkyl fatty acid alkanolamide, and alkyl polyglucoside, all of which are composed of fatty acid having 12 to 22 carbon atoms or an alkyl group having 12 to 22 carbon atoms.

Among those mentioned above, from the viewpoint of foam qualities and the volume of foam, glycerin fatty acid ester, polyoxyethylene alkyl ether, alkyl glyceryl ether, and alkyl polyglucoside are preferable.

Moreover, alkyl glyceryl ether is further preferable since rinsing properties are also improved.

One or more components (C) can be used, and from the viewpoint of the volume of foam, the content thereof is preferably 0.1% by mass or more, more preferably 0.5% by mass or more, even more preferably 1% by mass or more and preferably 30% by mass or less, more preferably 20% by mass or less, and even more preferably 15% by mass or less of the total composition. Further, the content thereof is preferably from 0.1 to 30% by mass, more preferably from 0.5 to 20% by mass, and even more preferably from 1 to 15% by mass of the total composition.

The skin cleansing composition of the present invention can further comprise water as a solvent. Water is added as balance of the cleansing composition other than the aforementioned components and other components composing the cleansing composition. The content of water is preferably 10% by mass or more, more preferably 15% by mass or more and preferably 94.5% by mass or less, and more preferably 90% by mass or less of the total composition.

As a favorable embodiment, the skin cleansing composition of the present invention preferably comprises the following components (A) and (B):

(A) an alkyl ether carboxylic acid or a salt thereof represented by formula (1):

$$R^1\text{-O-}(CH_2CH_2O)_n\text{-}CH_2\text{-COOM} \qquad (1)$$

wherein, $R^1$ represents an alkyl group hazing 10 to 16 carbon atoms, n represents a number of from 0 to 20, and KS1208 E(F) 121913 M represents a hydrogen atom, alkali metal, alkaline earth metal, ammonium, or organic ammonium,

wherein, $R^1$ has an average carbon number of from 10.8 to 12.8, n represents a number of from 0 to 20, and an average value of n is from 2.8 to 3.4,

and wherein, the alkyl ether carboxylic acid or a salt thereof contains a component in which n = 0 in an amount of 9.9% by mass or more and less than 12% by mass, preferably from 9.9 to 11.5% by mass, and the total content of a component in which n = 1 and a component in which n = 2 is from 20 to 36.5% by mass, and a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52, preferably 1 : 1.58 to 1.84 : 1.72 to 2.17 : 1.49 to 2.00 : 1.14 to 1.52, and

(B) an alkyl sulfate or polyoxyethylene alkyl sulfate represented by formula (2):

$$R^2\text{-O-}(CH_2CH_2O)_m\text{-}SO_3Y \qquad (2)$$

wherein, $R^2$ represents an alkyl group or an alkenyl group having 8 to 22 carbon atoms, m represents a number of from 0 to 20 and an average of m is 2 or more and less than 4, and Y represents a hydrogen atom or a cation selected from alkali metal, alkaline earth metal, ammonium, alkyl ammonium, alkanol ammonium, and glucammonium.

As an another preferable embodiment, the skin cleansing composition of the present invention preferably comprises the following components (A) and (B):

(A) an alkyl ether carboxylic acid or a salt thereof represented by formula (1):

$$R^1\text{-O-}(CH_2CH_2O)_n\text{-}CH_2\text{-COOM} \qquad (1)$$

wherein, $R^1$ represents an alkyl group having 10 to 16 carbon atoms, n represents a number of from 0 to 20, and M represents a hydrogen atom, alkali metal, alkaline earth metal, ammonium, or organic ammonium,

wherein, $R^1$ has an average carbon number of from 10.8 to 12.8, n represents a number of from 0 to 20, and an average value of n is from 2.8 to 3.4,

and wherein, the alkyl ether carboxylic acid or a salt thereof contains a component in which n = 0 in an amount of 12% by mass or more and 17% by mass or less, preferably from 13 to 17% by mass % by mass, and the total content of a component in which n = 1 and a component in which n = 2 is from 20 to 36.5% by mass, and a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99, preferably 1 : 1.00 to 1.31 : 0.93 to 1.34 : 0.79 to 1.18 : 0.63 to 0.99, and

(B) an alkyl sulfate or polyoxyethylene alkyl sulfate represented by formula (2):

$$R^2\text{-}O\text{-}(CH_2CH_2O)_m\text{-}SO_3Y \qquad (2)$$

wherein, $R^2$ represents an alkyl group or an alkenyl group having 8 to 22 carbon atoms, m represents a number of from 0 to 20 and an average of m is 2 or more and less than 4, and Y represents a hydrogen atom or a cation selected from alkali metal, alkaline earth metal, ammonium, alkyl ammonium, alkanol ammonium, and glucammonium.

The skin cleansing composition of the present invention may further comprise components used in ordinary cleansers such as surfactants other than the components (A), (B), and (C), humectants, oil components, disinfecting agents, anti-inflammatory agents, preservatives, chelating agents, thickening agents, salts, pearlescent agents, scrub agents, fragrances, cooling agents, dyes, ultraviolet absorbers, antioxidants, and plant extracts.

The skin cleansing composition of the present invention is produced by mixing the blending components by a routine method. The cleansing composition thus obtained may be either a liquid or a solid; however, when it is a liquid, the viscosity at 25°C as measured by a B-type viscometer (manufactured by Tokyo Keiki Inc.) is preferably from 200 to 80000 mPa·s. The viscosity can be adjusted by appropriately selecting the blending components.

Also, the pH is preferably from 3 to 12, more preferably from 5 to 10.5, even more preferably from 5 to 7. Also, the degree of pH is measured in each cleansing composition diluted 20-fold with ion exchange water at 25°C.

The skin cleansing composition of the present invention is suitably used as, for example, a face wash, a body soap, a hand soap, and the like. The skin cleansing composition of the present invention is more preferably used as a body soap.

A method for cleansing skin using the skin cleansing composition of the present invention is exemplified as follows. That is, a method including applying an adequate amount of the skin cleansing composition of the present invention to the body, namely the body's skin areas such as face, hands, feet, and torso, lathering up and washing, and then rinsing off using warm water from a shower and the like, is possible. It is also possible to apply an adequate amount of the cleansing composition of the present invention to a washing aid such as a towel, a sponge, and a brush, and then lather up and wash.

In connection with the aforementioned embodiments, the present invention further discloses the following compositions.

<1> A skin cleansing composition, comprising the following components (A) and (B) :

(A) an alkyl ether carboxylic acid or a salt thereof represented by formula (1):

$$R^1\text{-}O\text{-}(CH_2CH_2O)_n\text{-}CH_2\text{-}COOM \qquad (1)$$

wherein, $R^1$ represents an alkyl group having 10 to 16 carbon atoms, n represents a number of from 0 to 20, and M represents a hydrogen atom, alkali metal, alkaline earth metal, ammonium, or organic ammonium, wherein, $R^1$ has an average carbon number of from 10.8 to 12.8, and wherein, the alkyl ether carboxylic acid or a salt thereof contains a component in which n = 0 in an amount of more than 9.6% by mass and 27% by mass or less, and a component in which n = 1 and a component in which n = 2 in a total amount of 21% by mass or more and less than 40% by mass, and
(B) an alkyl sulfate or polyoxyethylene alkyl sulfate represented by formula (2):

$$R^2\text{-}O\text{-}(CH_2CH_2O)_m\text{-}SO_3Y \qquad (2)$$

wherein, $R^2$ represents an alkyl group or an alkenyl group having 8 to 22 carbon atoms, m represents a number of from 0 to 20 and an average of m is 2 or more and less than 4, and Y represents a hydrogen atom or a cation selected from alkali metal, alkaline earth metal, ammonium, alkyl ammonium, alkanol ammonium, and glucammonium.

<2> The skin cleansing composition according to the aforementioned <1>, wherein, in the component (A), in formula (1), $R^1$ represents an alkyl group having 10 to 16 carbon atoms and has an average carbon number of from 10.8 to 12.5, preferably from 12.1 to 12.4.

<3> The skin cleansing composition according to the aforementioned <1> or <2>, wherein, in the component (A), in formula (1), $R^1$ contains two or more alkyl groups and the content of a component having an alkyl chain length which is contained in the highest content is 55% by mass or more and less than 97% by mass, preferably from 60 to 95% by mass, and more preferably from 70 to 95% by mass.

<4> The skin cleansing composition according to any one of the aforementioned <1> to <3>, wherein, in the component (A), in formula (1), an average number of moles of ethylene oxide added (an average value of n) in a composition of the component (A) is from 2.8 to 3.4, and further preferably from 2.8 to 3.1.

<5> The skin cleansing composition according to any one of the aforementioned <1> to <4>, wherein the component (A) contains, in formula (1), a component in which n = 0 in an amount of from 9.8 to 27% by mass, preferably from 9.9 to 27% by mass, more preferably from 9.9 to 16% by mass, and even more preferably from 9.9 to 15% by mass.

<6> The skin cleansing composition according to any one of the aforementioned <1> to <5>, wherein the component (A) contains, in formula (1), a component in which n = 1 and a component in which n = 2 in a total amount of 21% by mass or more and less than 40% by mass, preferably from 27 to 37% by mass, more preferably from 27 to 36.5% by mass, and even more preferably from 35 to 36.1% by mass.

<7> The skin cleansing composition according to any one of the aforementioned <1> to <6>, wherein, in the component (A), in formula (1), a mass ratio of the components in which n = 0, 1, 2, 3, and 4 is (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52.

<8> The skin cleansing composition according to any one of the aforementioned <1> to <7>, wherein, the component (A) contains, in formula (1), a component in which n = 0 in an amount of 9.9% by mass or more and less than 12% by mass, and a ratio of (the mass of a component in which n = 0) : (the mass of a component in which n = 1) : (the mass of a component in which n = 2) : (the mass of a component in which n = 3) : (the mass of a component in which n = 4) = 1 : 1.53 to 1.87 : 1.59 to 2.25 : 1.33 to 2.16 : 1.14 to 1.52, or contains a component in which n = 0 in an amount of 12% by mass or more and 17% by mass or less, and a ratio of (the mass of a component in which n = 0) : (the mass of a component in which n = 1) : (the mass of a component in which n = 2) : (the mass of a component in which n = 3) : (the mass of a component in which n = 4) = 1 : 0.99 to 1.34 : 0.89 to 1.40 : 0.76 to 1.23 : 0.63 to 0.99.

<9> The skin cleansing composition according to any one of the aforementioned <1> to <8>, wherein the content of the component (A) is 0.1% by mass or more, preferably 0.5% by mass or more, and more preferably 1% by mass or more, and 30% by mass or less, preferably 20% by mass or less, and more preferably 15% by mass or less of a total composition.

<10> The skin cleansing composition according to any one of the aforementioned <1> to <9>, wherein, in the component (B), in formula (2), an average number of moles of ethylene oxide added (an average value of m) is 2 or more and less than 4, preferably from 2 to 3.5.

<11> The skin cleansing composition according to the aforementioned <1> or <10>, wherein the content of the component (B) is 0.1% by mass or more, preferably 0.5% by mass or more, more preferably 1% by mass or more and 30% by mass or less, preferably 20% by mass or less, and more preferably 15% by mass or less of a total composition.

<12> The skin cleansing composition according to any one of the aforementioned <1> to <11>, wherein a mass ratio of the component (A) to the component (B), (A) : (B), is from 1 : 10 to 10 : 1.

<13> The skin cleansing composition according to any one of the aforementioned <1> to <11>, wherein a mass ratio of the component (A) to the component (B), (A) : (B), is from 1 : 5 to 5 : 1.

<14> The skin cleansing composition according to any one of the aforementioned <1> to <11>, wherein a mass ratio of the component (A) to the component (B), (A) : (B), is from 1 : 2 to 2 : 1.

<15> The skin cleansing composition according to any one of the aforementioned <1> to <14>, further comprising (C) a nonionic surfactant.

<16> The skin cleansing composition according to the aforementioned <15>, wherein the nonionic surfactant of the component (C) is one or two or more selected from glycerin fatty acid ester, propylene glycol fatty acid ester, sorbitan fatty acid ester, polyoxyethylene sorbitan fatty acid ester, polyoxyethylene alkyl ether, polyoxyethylene polyoxypropylene glycol, alkyl glyceryl ether, polyethylene glycol fatty acid ester, alkyl alkanolamide, alkyl fatty acid alkanolamide, and alkyl polyglucoside, all of which are composed of fatty acid having 12 to 22 carbon atoms or an alkyl group having 12 to 22 carbon atoms.

<17> The skin cleansing composition according to the aforementioned <15>, wherein the nonionic surfactant of the component (C) is one or two or more selected from glycerin fatty acid ester, polyoxyethylene alkyl ether, alkyl glyceryl ether, and alkyl polyglucoside.

<18> The skin cleansing composition according to any one of the aforementioned <14> to <17>, wherein the content of the nonionic surfactant of the component (C) is 0.1% by mass or more, preferably 0.5% by mass or more, and more preferably 1% by mass or more, and is 30% by mass or less, preferably 20% by mass or less, and more preferably 15% by mass or less of a total composition.

<19> The skin cleansing composition according to any one of the aforementioned <1> to <18>, wherein the total content of the components (A) and (B) is 0.5% by mass or more, preferably 1% by mass or more, more preferably 5% by mass or more, and is 40% by mass or less, preferably 30% by mass or less, and more preferably 20% by mass or less of a total composition.

<20> A method for cleansing skin, comprising applying the skin cleansing composition according to any one of the aforementioned <1> to <19> to a skin area, washing, and then rinsing.

Examples

The alkyl ether carboxylate of the component (A) used in the skin cleansing composition of the present invention can be produced, for example, as follows.

Unless otherwise noted, "%" represents % by mass. Also, the alkyl composition, the distribution of the moles of EO added, and the ratio of each component of the alkyl ether carboxylic acid in a reaction mixture produced by the reaction were measured by the following analytical method with gas chromatography (GC).

(GC analytical conditions)

GC instrument; the product of Agilent Technologies, 7890A Column; the product of Agilent Technologies, DB-5 (30 m, an inner diameter of 0.25 mm, a film thickness of 0.25 $\mu$m)
Detector; FID
Carrier; helium gas, 1 mL/min
Conditions of temperature rising; temperature is raised at 5°C/min from 100°C to 325°C, and thereafter, maintained at 325°C for 35 minutes.

(Method of sample pretreatment)

Into 50 mL of methanol, 150 mg of alkyl ether carboxylate was dissolved. Also, the cleansing composition was taken in an amount of 150 mg in terms of alkyl ether carboxylate equivalent and dissolved in 50 mL of methanol. Also, when the cleansing composition contained a strong anionic surfactant such as polyoxyethylene alkyl ether sulfate, the cleansing composition was collected in such an amount that the strong anionic surfactant was 250 mg or less. From this solution, 1 mL was taken and applied to a solid phase cartridge (manufactured by Biotage Japan Ltd., Isolute SAX, 1 g, 3 mL, 500-0100-B) which had been conditioned with 4 mL of methanol in advance, and the filtrate was received in a 10 mL round-bottom test tube. Subsequently, the filtrate was eluted with 6 mL of a solution of 4.6 g of formic acid in 100 mL of methanol, and the eluate was also collected in the same test tube. The solution thus collected was set in a block heater heated to 50°C, to which nitrogen gas was blown in, and the solution was concentrated to approximately 1 mL, which was then dried at room temperature by further blowing nitrogen gas. To the resulting product, 2 mL of a diazomethane-ether solution was added, and the resulting solution was left to stand at room temperature for 10 minutes while stirring to carry out derivatization. Subsequently, nitrogen gas was blown in at room temperature and the solution was concentrated to 500 $\mu$L or less, to which chloroform was then added to bring the total volume to 500 $\mu$L, and the resulting product was subjected to GC analysis.

It is to be noted that the diazomethane-ether solution was prepared by the following procedure using a diazomethane generator (manufactured by Miyamoto Riken Ind. Co., Ltd., GM-50). A first receiver and a second receiver, and the second receiver and a third receiver were connected using a silicone rubber plug and a Teflon (Registered trademark) tube. Into the second receiver, 0.8 g of N-methyl-N'-nitro-N-nitrosoguanidine was collected, to which 2.5 mL of ion exchange water was added. Into the third receiver, 10 mL of tert-butyl methyl ether was collected. The first, second, and third receivers were cooled on ice. Subsequently, the second receiver was fitted with a plastic syringe, into which 3 mL of a solution of 20 g of sodium hydroxide dissolved in 100 mL of ion exchange water was placed. This aqueous solution of sodium hydroxide was slowly added dropwise to generate diazomethane gas, and nitrogen gas was gently blown in from the first receiver side to dissolve the diazomethane gas in tert-butyl methyl ether in the third receiver, whereby a diazomethane-ether solution was obtained.

The following reagents were used in the aforementioned sample pretreatment. Methanol (manufactured by Kanto Chemical Co., Inc., for high performance liquid chromatography, 25183-1B) Formic acid (manufactured by Wako Pure Chemical Industries, Ltd., special grade chemical, 066-00461) Chloroform (manufactured by Kanto Chemical Co., Inc., CICA first grade, 07278-01) N-Methyl-N'-nitro-N-nitrosoguanidine (manufactured by Kanto Chemical Co., Inc., CICA first grade, 25596-51) Tert-butyl methyl ether (manufactured by Kanto Chemical Co., Inc., CICA special grade, 04418-00) Sodium hydroxide (manufactured by Wako Pure Chemical Industries, Ltd., special grade, 196-13761)

Production Example 1

Into a stainless-steel autoclave equipped with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.68 g (0.0478 mol) of potassium hydroxide were placed and dehydration was performed under reduced pressure. Subsequently, 996 g (22.6 mol) of ethylene oxide (EO) was introduced at 155°C and reactions were allowed to proceed at a reaction temperature of 155°C and a reaction pressure of 0.4 MPa for two hours. Upon completion of the reaction, the resulting mixture was stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 3.55 moles of EO added (hereinbelow, also referred to as "the produced AE") was obtained.

Into a glass reaction container equipped with stirring and temperature controlling functions and an oxygen gas introduction tube, 90 g (0.2 mol) of the aforementioned product, 16.7 g of a 48% aqueous solution of sodium hydroxide (0.2 mol as sodium hydroxide), 0.9 g of a palladium-platinum-bismuth-based catalyst (activated carbon containing 4% of palladium, 1% of platinum, 5% of bismuth, and 50% of water), and 494.4 g of water were each placed. While stirring, the liquid temperature was raised to 70°C, and while blowing oxygen in at a ratio of 27 mol% (with respect to the produced AE / hour), catalytic oxidation reactions were allowed to proceed at a reaction temperature of 70°C for 3.5 hours. The rate of reaction was 89%.

Upon completion of the reaction, the catalyst was filtered out from the reaction solution to provide an aqueous solution of sodium salt of alkyl ether carboxylic acid. Subsequently, 35% hydrochloric acid was added, and a liquid separation operation was performed to provide alkyl ether carboxylic acid, which will be referred to as EC1.

As a result of gas chromatography analysis, it was found that, in formula (1), M = H, $R^1$ had lauryl group / myristyl group at a ratio of 95 / 5, the average carbon number was 12.1, and the average value of n was 2.8, and EC1 contained a component in which n = 0 in an amount of 14.7% by mass, and a component in which n = 1 and a component in which n = 2 in a total amount of 36.1% by mass.

Further, it was also found that the ratio of each of the components having different numbers of moles of EO added, as calculated from the measurement value of the maximum component of the composition of $R^1$, was as follows; (the mass of a component in which n = 0) : (the mass of a component in which n = 1) : (the mass of a component in which n = 2) : (the mass of a component in which n = 3) : (the mass of a component in which n = 4) = 1 : 1.22 : 1.23 : 1.06 : 0.83.

Production Example 2

According to Production Example 1, EO was reacted with a raw material containing a mixture of decyl alcohol [trade name: KALCOL 1098, manufactured by Kao Corporation], lauryl alcohol [trade name: KALCOL 2098, manufactured by Kao Corporation], myristyl alcohol [trade name: KALCOL 4098, manufactured by Kao Corporation], and cetyl alcohol [trade name: KALCOL 6098, manufactured by Kao Corporation] at a mass ratio of 10 / 70 / 15 / 5 to provide alkyl ethoxylate having 3.55 moles of EO added. In the same manner as in Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in formula (1), M = H, $R^1$ had decyl group / lauryl group / myristyl group / palmityl group at a ratio of 10 / 70 / 15 / 5, the average carbon number was 12.3, and the average value of n was 3.3, and the alkyl ether carboxylic acid contained a component in which n = 0 in an amount of 15.2% by mass, and a component in which n = 1 and a component in which n = 2 in a total amount of 31.4% by mass.

Further, it was also found that the ratio of each of the components having different numbers of moles of EO added, as calculated from the measurement value of the maximum component of the composition of $R^1$, was as follows; (the mass of a component in which n = 0) : (the mass of a component in which n = 1) : (the mass of a component in which n = 2) : (the mass of a component in which n = 3) : (the mass of a component in which n = 4) = 1 : 1.07 : 1.00 : 0.85 : 0.67.

Production Example 3

Into a glass reaction container equipped with stirring and temperature controlling functions, 372 g (2.00 mol) of lauryl alcohol was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, the precipitates were filtered out. Subsequently, 35% hydrochloric acid was added for acidification to obtain alkyl ether carboxylic acid (in formula (1), M = H, $R^1$ was a lauryl group, and n = 0).

Production Example 4

According to Production Example 1, EO was reacted with decyl alcohol as a raw material to provide alkyl ethoxylate having 3.55 moles of EO added. In the same manner as in Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in formula (1), M = H, $R^1$ was a decyl group, and the average value of n was 3.1, and the alkyl ether carboxylic acid contained a component in which n = 0 in an amount of 16% by mass, and a component in which n = 1 and a component in which n = 2 in a total amount of 37% by mass.

Production Example 5

According to Production Example 1, EO was reacted with lauryl alcohol as a raw material to provide alkyl ethoxylate having 3.55 moles of EO added. In the same manner as in Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in formula (1), M = H, $R^1$ was a lauryl group, and the average value of n was 3.1, and the alkyl ether carboxylic acid contained a component in which n = 0 in an amount of 16% by mass, and a component in which n = 1 and a component in which n = 2 in a total amount of 37% by mass.

Production Example 6

According to Production Example 1, EO was reacted with myristyl alcohol as a raw material to provide alkyl ethoxylate having 3.55 moles of EO added. In the same manner as in Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in formula (1), M = H, $R^1$ was a myristyl group, and the average value of n was 3.1, and the alkyl ether carboxylic acid contained a component in which n = 0 in an amount of 16% by mass, and a component in which n = 1 and a component in which n = 2 in a total amount of 37% by mass.

Production Example 7

According to Production Example 1, EO was added to a raw material containing a mixture of lauryl alcohol and cetyl alcohol at a mass ratio of 20 / 80 to provide alkyl ethoxylate having 3.55 moles of EO added. In the same manner as in Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in formula (1), M = H, $R^1$ had lauryl group / palmityl group at a ratio of 20 / 80, and the average value of n was 3.1, and the alkyl ether carboxylic acid contained a component in which n = 0 in an amount of 16% by mass, and a component in which n = 1 and a component in which n = 2 in a total amount of 37% by mass.

Production Example 8

According to Production Example 1, EO was reacted with lauryl alcohol as a raw material to provide alkyl ethoxylate having 4.05 moles of EO added. In the same manner as in Production Example 1, the alkyl ethoxylate thus obtained was subjected to an oxidation reaction, and the resulting alkyl ether carboxylate was subjected to hydrochloric acid treatment, whereby alkyl ether carboxylic acid was obtained.

As a result of gas chromatography analysis, it was found that, in formula (1), M = H, $R^1$ was a lauryl group, the average value of n was 3.5, and the resulting alkyl ether carboxylic acid contained a component in which n = 0 in an amount of 11.4% by mass, and a component in which n = 1 and a component in which n = 2 in a total amount of 30.6% by mass.

Further, it was also found that the ratio of each of the components having different numbers of moles of EO added, as calculated from the measurement value of the maximum component of the composition of $R^1$, was as follows; (the mass of a component in which n = 0) : (the mass of a component in which n = 1) : (the mass of a component in which n = 2) : (the mass of a component in which n = 3) : (the mass of a component in which n = 4) = 1 : 1.31 : 1.38 : 1.25 : 1.06.

Production Example 9

Into a stainless-steel autoclave equipped with stirring and temperature controlling functions, 1144 g (6.14 mol) of lauryl alcohol [trade name: KALCOL 2098, manufactured by Kao Corporation], 60.2 g (0.281 mol) of myristyl alcohol [trade name: KALCOL 4098, manufactured by Kao Corporation], and 2.6 g (0.0478 mol) of potassium hydroxide were placed and dehydration was performed under reduced pressure. Subsequently, 718 g (16.3 mol) of ethylene oxide (EO) was introduced at 155°C and a reaction was allowed to proceed at a reaction temperature of 155°C and a reaction pressure of 0.4 MPa for two hours. Upon completion of the reaction, the resulting mixture was cooled and then stirred for 30 minutes at 80°C under a reduced pressure condition of 6 kPa. Then, after removing unreacted ethylene oxide, nitrogen was introduced to normalize the pressure, and 4.82 g (0.0482 mol) of 90% lactic acid was added into the autoclave, followed by stirring at 80°C for 30 minutes, whereby alkyl ethoxylate having 2.55 moles of EO added was obtained.

Into a glass reaction container equipped with stirring and temperature controlling functions, 600 g (2.00 mol) of the aforementioned product was placed, and while stirring, the liquid temperature was raised to 70°C. Then, while adding 256 g (2.20 mol) of sodium monochloroacetate and 88 g (2.20 mol) of sodium hydroxide in divided portions, a reaction was allowed to proceed for five hours. Upon completion of the reaction, 35% hydrochloric acid was added for acidification until pH was 2.8, and the resulting oil layer was collected to obtain alkyl ether carboxylic acid, which will be referred to as EC6.

As a result of gas chromatography analysis, it was found that, in formula (1), M = H, $R^1$ had lauryl group / myristyl group at a ratio of 94 / 6, the average carbon number was 12.1, and the average value of n was 3.1, and EC6 contained a component in which n = 0 in an amount of 9.9% by mass, and a component in which n = 1 and a component in which n = 2 in a total amount of 35.4% by mass.

Further, it was also found that the ratio of each of the components having different numbers of moles of EO added, as calculated from the measurement value of the maximum component of the composition of $R^1$, was as follows; (the mass of a component in which n = 0) : (the mass of a component in which n = 1) : (the mass of a component in which n = 2) : (the mass of a component in which n = 3) : (the mass of a component in which n = 4) = 1 : 1.65 : 1.92 : 1.74 : 1.32.

In the Examples, EC2 was obtained by mixing each of the alkyl ether carboxylic acids produced in Production Examples 5, 6, and 7 at a mass ratio of 78.75 / 15 / 6.25.

In the Examples, EC3 was obtained by mixing each of the alkyl ether carboxylic acids obtained in Production Examples 2 and 3 at a mass ratio of 90 / 10.

In the Examples, EC4 was obtained by mixing each of EC1 obtained in Production Example 1 and the alkyl ether carboxylic acid obtained in Production Example 4 at a mass ratio of 40 / 60.

In the Examples, EC5 was obtained by mixing each of the alkyl ether carboxylic acids obtained in Production Examples 2 and 8 at a mass ratio of 40 / 60.

In the Examples, EC7 was obtained by mixing each of EC1 obtained in Production Example 1 and commercially available AKYPO RLM45 (manufactured by Kao Corporation) at a mass ratio of 50 / 50.

In the Examples, EC8 was obtained by mixing each of EC1 obtained in Production Example 1 and commercially available AKYPO RLM100NV (manufactured by Kao Corporation) at a mass ratio of 50 / 50.

Examples 1 to 35 and Comparative Examples 1 to 14

The skin cleansing compositions having the compositions as shown in Tables 3 to 5 were produced, and the volume of foam, ease of foam dripping, foam qualities, and rinsing properties were evaluated. Also, for evaluation of mildness to the skin, the amount of Zein dissolved was obtained, and further, easiness of spreading of foam was evaluated. The results are shown in Tables 3 to 5 altogether.

Also, the composition of the component (A) used in Examples is as shown in Tables 1 and 2.

Also, for the average numbers of moles of EO added of commercially available alkyl ether carboxylic acids used in Examples (AKYPO RLM25 (manufactured by Kao Corporation), AKYPO RLM45 (manufactured by Kao Corporation), AKYPO RLM100NV (manufactured by Kao Corporation), BEAULIGHT LCA (manufactured by Sanyo Chemical Industries, Ltd.), and ECTD-3NEX (manufactured by Nikko Chemicals, Co., Ltd)), values provided in the catalogue supplied by each vendor or values posted in the website of each vendor were referred to. Unknown alkyl composition, the amount of a component in which n = 0, and the total amount of a component in which n = 1 and a component in which n = 2 were analyzed by the aforementioned method.

(Production method)

Each component (except sodium hydroxide) was mixed with water and homogenized while heating to 70°C.

Subsequently, sodium hydroxide was added in an amount to bring pH to 6, followed by stirring to homogeneity. Further, after cooling to room temperature while stirring, whereby skin cleansing compositions were obtained.

(Evaluation method)

(1) Volume of foam:

A lathering mesh (manufactured by Daisan Co. of Hakugen Group) was moistened with water (approximately 8 g). Then, 1 g of each skin cleansing composition was placed on the lathering mesh. The lathering mesh was held in both hands in an enveloping manner and the hands were moved in a circular motion for lathering. The hands were rubbed together in a circular motion 40 times for lathering. The foam produced with the lathering mesh was collected in a 500 mL beaker (manufactured by AGC Techno Glass Co., Ltd., 8.5 cm in diameter and 15 cm in height). After condensing the foam by shaking the beaker, the height (cm) of the foam thus collected is measured with a ruler. Then, the volume ($cm^3$) of the foam thus collected was calculated from the height thus obtained and the area of the base of the beaker.

(2) Ease of foam dripping:

Approximately 50 mL of the foam produced in (1) is placed on forearm wet with water (approximately 3 cm from the wrist). Subsequently, the foam-placed forearm was held up perpendicularly to the ground. After maintaining the perpendicular position for 30 seconds, the distance of dripping of the foam from the initial position was measured. This operation was repeated three times and an average value of three operations was obtained, which was used as ease of foam dripping.

(3) Foam qualities:

One expert panelist organoleptically evaluated the foam qualities of the foam produced in (1) based on the following criteria.

5; Foam is very fine and soft.
4; Foam is slightly fine and soft.
3; Foam is soft.
2; Foam is slightly large, fragile, and slightly watery.
1; Foam is large, fragile, and watery.

(4) Rinsing properties:

After evaluation of ease of foam dripping (2), the foam was spread over the entire forearm and rubbed 10 times in a back and forth motion to wash the arm, followed by rinsing with tap water (approximately 30°C). At this time, the rinsing properties were organoleptically evaluated by one expert panelist based on the following criteria.

5; A slimy feel rapidly disappears, immediately followed by an appropriate feeling of friction.
4; A slimy feel slightly rapidly disappears, followed by an appropriate feeling of friction.
3; A slimy feel disappears, followed by a feeling of friction.
2; It takes some time for a slimy feel to disappear during rinsing, followed by a weak feeling of friction.
1; It took a long time for a slimy feel to disappear during rinsing, followed by a very weak feeling of friction.

(5) Mildness to the skin (amount of Zein dissolved):

In a sample vial (manufactured by Maruemu Corporation, No. 7 screw vial), 6 g of each skin cleansing composition was placed, followed by dilution with 24 g of ion exchanged water. To the resulting mixture, 3 g of Zein (Zein from maize, manufactured by Sigma-Aldrich) was added, followed by vigorous stirring for one hour with a magnetic stirrer. When Zein was completely dissolved while stirring, more Zein was added until undissolved Zein remained. Subsequently, undissolved Zein was filtered out and collected on filter paper (Toyo Roshi 5A 125 mm) which was cut out approximately 5 cm in diameter. Further, liquids were removed by suction filtration. Subsequently, Zein on the filter paper was dried under vacuum at 50°C for eight hours to further remove moisture. Then, the dried filter paper and Zein were weighed and the amount of Zein dissolved was obtained by the following formula.
The smaller the amount of Zein dissolved, the milder the product to the skin.

```
        Amount of Zein dissolved (g)

        = Total amount of Zein added (g) - (weight of dried

    filter paper and Zein (g) - weight of filter paper (g))
```

(6) Ease of spreading of foam:

Each skin cleansing composition (6 g) was lathered with a nylon towel (manufactured by Marna, Inc., material: 100% nylon, size: 30 cm x 105 cm) and then used for washing the whole body. The "ease of spreading of foam" attributable to the volume of foam, ease of foam dripping, and foam qualities were evaluated based on the following criteria.

A; Foam is very easily spread (foam is abundant, soft, and difficultly drips off).
B; Foam is easily spread (an appropriate amount of foam having soft foam qualities, which is difficult to drip off).
C; Foam does not easily spread out.
D; Due to the watery and bubbly foam qualities, the foam easily drips off the skin.

[Table 1]

| | R[1] (% by mass) | | | | Average carbon number | Average number of moles of EO added | n=0 Content ratio | n=1,2 Total amount | M (Salt) |
|---|---|---|---|---|---|---|---|---|---|
| | C10 | C12 | C14 | C16 | | | | | |
| EC1 | 0 | 95 | 5 | 0 | 12.1 | 2.8 | 14.7% | 36.1% | Na |
| EC2 | 0 | 80 | 15 | 5 | 12.5 | 3.1 | 16.0% | 32.5% | Na |
| EC3 | 8 | 73 | 13.5 | 4.5 | 12.2 | 2.8 | 27.0% | 27.1% | Na |
| EC4 | 60 | 38 | 2 | 0 | 10.8 | 3.2 | 12.5% | 34.8% | Na |
| EC5 | 4 | 88 | 6 | 2 | 12.1 | 3.4 | 13.3% | 31.0% | Na |
| EC6 | 0 | 94 | 6 | 0 | 12.1 | 3.1 | 9.9% | 35.4% | Na |

[Table 2]

| | R[1] (% by mass) | | | | Average carbon number | Average number of moles of EO added | n=0 Content ratio | n=1, 2 Total amount | M (Salt) |
|---|---|---|---|---|---|---|---|---|---|
| | C10 | C12 | C14 | C16 | | | | | |
| 25CA *1 | 0 | 68 | 26 | 6 | 12.8 | 2.5 | 16.0% | 32.5% | Na |
| EC7 | 0 | 81.5 | 15.5 | 3 | 12.5 | 3.7 | 12.2% | 33.7% | Na |
| EC8 | 0 | 81.5 | 15.5 | 3 | 12.5 | 6.4 | 9.8% | 28.2% | Na |
| 45CA *2 | 0 | 68 | 26 | 6 | 12.8 | 4.5 | 9.6% | 31.2% | Na |
| 100NV *3 | 0 | 68 | 26 | 6 | 12.8 | 10.0 | 4.9% | 20.4% | Na |
| LCA *4 | 0 | 100 | 0 | 0 | 12.0 | 3.0 | 2.8% | 42.8% | Na |

(continued)

| | R$^1$(% by mass) | | | | Average carbon number | Average number of moles of EO added | n=0 Content ratio | n=1, 2 Total amount | M (Salt) |
|---|---|---|---|---|---|---|---|---|---|
| | C10 | C12 | C14 | C16 | | | | | |
| Nex *5 | - | - | - | - | C13 branched | 3.0 | 4.2% | 40.0% | Na |

| |
|---|
| *1: AKYPO RLM25 (manufactured by Kao Corporation) |
| *2: AKYPO RLM45 (manufactured by Kao Corporation) |
| *3: AKYPO RLM100NV (manufactured by Kao Corporation) |
| *4: BEALTLIGHT LCA (manufactured by Sanyo Chemical Industries, Ltd.) |
| *5: ECTD-3NEX (manufactured by Nikko Chemicals, Co., Ltd) |

[Table 3]

| Component (% by mass) | Example | | | | | | | | | | | Comparative Example | | | | | | | | | | | | | | | |
|---|---|---|---|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 1 | 2 | 3 | 4 | 5 | 6 | 7 | 8 | 9 | 10 | 11 | 12 | 13 | 14 | 15 | 16 |
| EC1 | 7.5 | | | | | | | | | 7.5 | | | | | | | | | | | | | | | | | |
| EC2 | | 7.5 | | | | | | | | | | | | | | | | | | | | | | | | | |
| EC3 | | | 7.5 | | | | | | | | | | | | | | | | | | | | | | | | |
| EC4 | | | | 7.5 | | | | | | | | | | | | | | | | | | | | | | | |
| EC5 | | | | | 7.5 | | | | | | | | | | | | | | | | | | | | | | |
| EC6 | | | | | | 7.5 | | | | | | | | | | | | | | | | | | | | | |
| 25CA *1 | | | | | | | 7.5 | | | | 7.5 | 15 | | | | | | | | | | | | | | | |
| EC7 | | | | | | | | 7.5 | | | | | 15 | | | | | | | | | | | | | | |
| EC8 | | | | | | | | | 7.5 | | | | | 15 | | | | | | | | | | | | | |
| 45CA *2 | | | | | | | | | | | | | | | 15 | | | | | 7.5 | | | | 7.5 | | |
| 100NV *3 | | | | | | | | | | | | | | | | 15 | | | | | 7.5 | | | | | |
| LCA *4 | | | | | | | | | | | | | | | | | 15 | | | | | 7.5 | | | 7.5 | |
| Nex *5 | | | | | | | | | | | | | | | | | | 15 | | | | | 7.5 | | | 7.5 |
| Sodium polyoxyethylene (2) lauryl ether sulfate *6 | 7.5 | 7.5 | 7.5 | 7.5 | 7.5 | 7.5 | 7.5 | 7.5 | 7.5 | | | | | | | | | | 15 | 7.5 | 7.5 | 7.5 | 7.5 | | | | |
| Sodium polyoxyethylene (3) lauryl ether sulfate *7 | | | | | | | | | | 7.5 | 7.5 | | | | | | | | | | | | | 7.5 | 7.5 | 7.5 | 15 |
| Aqueous solution of sodium hydroxide | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. |
| Purified water | Balance | Balance | Balance | Balance | Balance | Balance | Balance | Balance | Balance | Balance | Balance | Balance | Balance | Balance | Balance | Balance | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 |
| pH | 6 | 6 | 6 | 6 | 6 | 6 | 6 | 6 | 6 | 6 | 6 | 6 | 6 | 6 | 6 | 6 | 6 | 6 | 6 | 6 | 6 | 6 | 6 | 6 | 6 | 6 | 6 |
| Evaluation item: Volume of foam (cm3) | 425 | 397 | 454 | 482 | 454 | 400 | 369 | 340 | 340 | 340 | 284 | 255 | 255 | 227 | 284 | 284 | 397 | 510 | 425 | 284 | 284 | 340 | 510 | 284 | 255 | 510 | 397 |

| | | | | | | | | | | | | | | | | | | | | | | | | | | | |
|---|---|---|---|---|---|---|---|---|---|---|---|---|---|---|---|---|---|---|---|---|---|---|---|---|---|---|---|
| Ease of foam dripping (cm) | 7.7 | 5.7 | 8.0 | 5.7 | 5.7 | 7.0 | 6.7 | 8.3 | 7.3 | 8.0 | 7.3 | 6.7 | 5.7 | 0.3 | 0.3 | 1.3 | 1.7 | 0.3 | 0.3 | 0.0 | 1.7 | 0.0 | 0.3 | 0.3 | 0.3 | 0.0 | 0.0 |
| Foam qualities | 1.0 | 1.0 | 2.0 | 2.0 | 1.0 | 2.0 | 2.0 | 3.0 | 1.0 | 1.0 | 2.0 | 2.0 | 2.0 | 3.0 | 3.0 | 3.0 | 3.0 | 3.0 | 3.0 | 3.0 | 3.0 | 4.0 | 3.0 | 3.0 | 3.0 | 4.0 | 4.0 |
| Rinsing properties | 1.0 | 1.0 | 2.0 | 2.0 | 1.0 | 2.0 | 2.0 | 2.5 | 1.0 | 1.0 | 3.0 | 2.0 | 2.0 | 3.0 | 3.0 | 3.0 | 2.0 | 3.0 | 3.0 | 3.0 | 3.0 | 3.0 | 3.0 | 3.0 | 3.0 | 3.0 | 3.0 |
| Amount of Zein dissolved (g) | 1.2 | 0.6 | 0.5 | 0.2 | 0.8 | 0.8 | 0.3 | 0.4 | 1.5 | 0.9 | 0.8 | 0.3 | 0.7 | 0.7 | 0.8 | 0.4 | 0.9 | 1.0 | 0.9 | 1.0 | 0.4 | 0.5 | 0.4 | 0.5 | 0.6 | 1.0 | 0.6 |
| Ease of spreading of foam | D | D | D | D | D | D | D | D | D | D | D | D | D | C | C | C | B | A | A | A | A | A | A | A | A | A | A |

*1: AKYPO RLM25 (manufactured by Kao Corporation)
*2: AKYPO RLM45 (manufactured by Kao Corporation)
*3: AKYPO RLM100NV (manufactured by Kao Corporation)
*4: BEAULIGHT LCA (manufactured by Sanyo Chemical Industries, Ltd.)
*5: ECTD-3NEX (manufactured by Nikko Chemicals, Co., Ltd)
*6: EMAL 270J (manufactured by Kao Corporation)
*7: EMAL 327 (manufactured by Kao Corporation)

[Table 4]

| | Component (% by mass) | Example | | | | | | | | | | | | | | Comparative Example | |
|---|---|---|---|---|---|---|---|---|---|---|---|---|---|---|---|---|---|
| | | 12 | 13 | 14 | 15 | 16 | 17 | 18 | 19 | 20 | 21 | 22 | 23 | 24 | 25 | 13 | 14 |
| A | EC1 | 1.4 | 2.5 | 5.0 | 10.0 | 12.5 | 13.6 | 15 | 20 | 9.0 | 1.0 | 4.5 | 0.5 | 1.5 | | 3.0 | 0.0 |
| | EC6 | | | | | | | | | | | | | | 1.5 | | |
| B | Sodium polyoxyethylene (2) lauryl ether sulfate *6 | 13.6 | 12.5 | 10.0 | 5.0 | 2.5 | 1.4 | 15 | 20 | 1.0 | 9.0 | 0.5 | 4.5 | 1.5 | 1.5 | 0.0 | 3.0 |
| | Aqueous solution of sodium hydroxide | q.s. | q.s. | q.s. | q.s. | q.s. | q.s. | q.s. | q.s. | q.s. | q.s. | q.s. | q.s. | q.s. | q.s. | q.s. | q.s. |
| | Purified water | Balance | Balance | Balance | Balance | Balance | Balance | Balance | Balance | Balance | Balance | Balance | Balance | Balance | Balance | Balance | Balance |
| | Total | 100 | 100 | 100 | 100 | 100 | 100 | 100 | 100 | 100 | 100 | 100 | 100 | 100 | 100 | 100 | 100 |
| | pH | 6 | 6 | 6 | 6 | 6 | 6 | 6 | 6 | 6 | 6 | 6 | 6 | 6 | 6 | 6 | 6 |
| | (A):(B) | 1:10 | 1:5 | 1:2 | 2:1 | 5:1 | 10:1 | 1:1 | 1:1 | 9:1 | 1:9 | 9:1 | 1:9 | 1:1 | 1:1 | 1:0 | 0:1 |
| | Evaluation item: Volume of foam (cm3) | 340 | 397 | 425 | 567 | 510 | 397 | 454 | 510 | 397 | 397 | 340 | 340 | 284 | 270 | 255 | 255 |
| | Ease of foam dripping(cm) | 0.7 | 0.3 | 0.0 | 0.0 | 0.0 | 0.3 | 0.0 | 0.0 | 0.0 | 0.3 | 0.0 | 0.3 | 0.0 | 0.0 | 0.0 | 5.7 |
| | Foam qualities | 3.0 | 4.0 | 4.0 | 4.0 | 4.0 | 3.0 | 4.0 | 4.0 | 3.0 | 3.0 | 3.0 | 3.0 | 3.0 | 3.0 | 2.0 | 1.0 |
| | Rinsing properties | 3.0 | 3.0 | 3.0 | 3.0 | 3.0 | 3.0 | 3.0 | 3.0 | 3.0 | 3.0 | 3.0 | 3.0 | 3.0 | 3.0 | 3.0 | 1.0 |
| | Amount of Zein dissolved (g) | 1.4 | 1.1 | 0.8 | 1.1 | 0.5 | 0.5 | 0.6 | 0.6 | 0.5 | 1.4 | 0.5 | 1.4 | 0.6 | 0.6 | 0.6 | 1.5 |

EP 2 752 186 B1

18

(continued)

| Component (% by mass) | Example | | | | | | | | | | | | | | Comparative Example | |
|---|---|---|---|---|---|---|---|---|---|---|---|---|---|---|---|---|
| | 12 | 13 | 14 | 15 | 16 | 17 | 18 | 19 | 20 | 21 | 22 | 23 | 24 | 25 | 13 | 14 |
| Ease of spreading of foam | A | A | A | A | A | A | A | A | A | A | A | A | B | A | C | D |
| *6: EMAL 270J (manufactured by Kao Corporation) | | | | | | | | | | | | | | | | |

[Table 5]

| | Component (% by mass) | Example | | | | | | | | | |
|---|---|---|---|---|---|---|---|---|---|---|---|
| | | 26 | 27 | 28 | 29 | 30 | 31 | 32 | 33 | 34 | 35 |
| A | EC1 | 5.0 | 5.0 | 5.0 | 5.0 | 2.5 | 5.0 | 5.0 | 5.0 | 5.0 | 5.0 |
| B | Sodium polyoxyethylene (2) lauryl ether sulfate *6 | 5.0 | 5.0 | 5.0 | 5.0 | 5.0 | 5.0 | 5.0 | 5.0 | 5.0 | 5.0 |
| C | Alkyl polyglucoside *9 | | 10.0 | | | 10.0 | 5.0 | | | | 14.5 |
| | 2-Ethylhexyl glyceryl ether *10 | 0.3 | | 2.5 | | 0.5 | | | 0.1 | 0.5 | 0.5 |
| | Polyoxyethylene (16) lauryl ether *11 | | | | | 1.0 | | | | | |
| | Polyoxyethylene (3) alkyl ether *12 | | | | 1.0 | | | | | | |
| | Glycerylcaprate *13 | | | | | 1.0 | | | | | |
| | Aqueous solution of sodium hydroxide | q.s. | q.s. | q.s. | q.s. | q.s. | q.s. | q.s. | q.s. | q.s. | q.s. |
| | Purified water | Balance | Balance | Balance | Balance | Balance | Balance | Balance | Balance | Balance | Balance |
| | Total | 100 | 100 | 100 | 100 | 100 | 100 | 100 | 100 | 100 | 100 |
| | pH | 6 | 6 | 6 | 6 | 6 | 6 | 6 | 6 | 6 | 6 |
| | (A):(C) | 1:15 | 1:2 | 2:1 | 5:1 | 5:1 | 1:1 | - | 50:1 | 10:1 | 1:3 |
| | Evaluation item: Volume of foam (cm3) | 450 | 500 | 450 | 440 | 510 | 480 | 400 | 430 | 480 | 550 |
| | Ease of foam dripping (cm) | 0.0 | 0.0 | 0.0 | 0.0 | 0.0 | 0.0 | 0.0 | 0.0 | 0.0 | 0.0 |
| | Foam qualities | 3.0 | 4.0 | 4.0 | 3.5 | 4.0 | 4.0 | 4.0 | 3.5 | 3.0 | 4.0 |
| | Rinsing properties | 3.5 | 3.0 | 3.0 | 3.0 | 3.0 | 3.0 | 3.0 | 3.0 | 4.0 | 3.5 |
| | Amount of Zein dissolved (g) | 0.6 | 0.6 | 0.6 | 0.6 | 0.6 | 0.6 | 0.6 | 0.6 | 0.6 | 0.6 |
| | Ease of spreading of foam | A | A | A | A | A | A | A | A | A | A |

*6: EMAL 270J (manufactured by Kao Corporation)
*9: AG-124 (manufactured by Kao Corporation)
*10: PENETOL GE-EH (manufactured by Kao Corporation)
*11: EMULGEN 116A (manufactured by Kao Corporation)
*12: SOFTANOL 33 (manufactured by Nippon Shokubai Co., Ltd.)
*13: SUNSOFT No. 760H (manufactured by Taiyo Kagaku Co., Ltd.)

EP 2 752 186 B1

**Claims**

1. A skin cleansing composition, comprising the following components (A) and (B):

   (A) an alkyl ether carboxylic acid or a salt thereof represented by formula (1) :

   $$R^1-O-(CH_2CH_2O)_n-CH_2-COOM \qquad (1)$$

   wherein, $R^1$ represents an alkyl group having 10 to 16 carbon atoms, n represents a number of from 0 to 20, and M represents a hydrogen atom, alkali metal, alkaline earth metal, ammonium, or organic ammonium, wherein, $R^1$ has an average carbon number of from 10.8 to 12.8, wherein, the alkyl ether carboxylic acid or a salt thereof contains a component in which n = 0 in an amount of 9.8 to 27% by mass, and a component in which n = 1 and a component in which n = 2 in a total amount of 21% by mass or more and less than 40% by mass, and an average value of n is from 2.8 to 3.4, and

   (B) a polyoxyethylene alkyl sulfate represented by formula (2) :

   $$R^2-O-(CH_2CH_2O)_m-SO_3Y \qquad (2)$$

   wherein, $R^2$ represents an alkyl group or an alkenyl group having 8 to 22 carbon atoms, m represents a number of from 0 to 20 and an average of m is 2 or more and less than 4, and Y represents a hydrogen atom or a cation selected from alkali metal, alkaline earth metal, ammonium, alkyl ammonium, alkanol ammonium, and glucammonium.

2. The skin cleansing composition according to claim 1, wherein, in the component (A), in the formula (1), an average value of n is from 1.5 to 10.

3. The skin cleansing composition according to any one of claims 1 to 2, wherein, in the component (A), in the formula (1), $R^1$ represents an alkyl group having 10 to 16 carbon atoms, an average carbon number of $R^1$ is from 10.8 to 12.5, the average value of n is from 2.8 to 3.4, a component in which n = 0 is contained in an amount of from 9.9 to 16% by mass, and a component in which n = 1 and a component in which n = 2 are contained in a total amount of from 27 to 36.5% by mass.

4. The skin cleansing composition according to any one of claims 1 to 3, wherein, in the component (A), in the formula (1), (the mass of a component in which n = 0) : (the mass of a component in which n = 1) : (the mass of a component in which n = 2) : (the mass of a component in which n = 3) : (the mass of a component in which n = 4) = 1 : 0.99 to 3.50 : 0.89 to 3.00 : 0.76 to 3.00 : 0.63 to 1.52.

5. The skin cleansing composition according to any one of claims 1 to 4, wherein, in the component (A), in the formula (1), $R^1$ contains two or more alkyl groups, and the content of a component having an alkyl chain length which is contained in the highest content is 55% by mass or more and less than 97% by mass.

6. The skin cleansing composition according to any one of claims 1 to 5, which comprises a component in which n = 0 in an amount of 9.9% by mass or more and less than 12% by mass and has a ratio of (the mass of a component in which n = 0) : (the mass of a component in which n = 1) : (the mass of a component in which n = 2) : (the mass of a component in which n = 3) : (the mass of a component in which n = 4) of 1 : 1.53 to 1.87 : 1.59 to 2.25 : 1.33 to 2.16 : 1.14 to 1.52, or comprises a component in which n = 0 in an amount of 12% by mass or more and 17% by mass or less and has a ratio of (the mass of a component in which n = 0) : (the mass of a component in which n = 1) : (the mass of a component in which n = 2) : (the mass of a component in which n = 3) : (the mass of a component in which n = 4) of 1 : 0.99 to 1.34 : 0.89 to 1.40 : 0.76 to 1.23 : 0.63 to 0.99, in the component (A), in the formula (1).

7. The skin cleansing composition according to any one of claims 1 to 6, wherein a mass ratio of the component (A) to the component (B), (A) : (B), is from 1 : 10 to 10 : 1.

8. The skin cleansing composition according to any one of claims 1 to 7, wherein a mass ratio of the component (A) to the component (B), (A) : (B), is from 1 : 5 to 5 : 1.

9. The skin cleansing composition according to any one of claims 1 to 8, wherein a mass ratio of the component (A) to the component (B), (A) : (B), is from 1 : 2 to 2 : 1.

10. The skin cleansing composition according to any one of claims 1 to 9, further comprising (C) a nonionic surfactant.

11. A method for cleansing skin, comprising applying the skin cleansing composition according to any one of claims 1 to 10 to a skin area, washing, and then rinsing.

12. Use of the cleansing composition according to any one of claims 1 to 11 as a skin cleansing agent.


**Patentansprüche**

1. Hautreinigungszusammensetzung, umfassend die folgenden Komponenten (A) und (B):

(A) eine Alkylethercarbonsäure oder ein Salz davon, dargestellt durch Formel (1):

$$R^1\text{-O-}(CH_2CH_2O)_n\text{-}CH_2\text{-COOM} \qquad (1),$$

wobei $R^1$ eine Alkylgruppe mit 10 bis 16 Kohlenstoffatomen darstellt, n eine Zahl von 0 bis 20 darstellt und M ein Wasserstoffatom, Alkalimetall, Erdalkalimetall, Ammonium oder organisches Ammonium darstellt, wobei $R^1$ eine durchschnittliche Kohlenstoffanzahl von 10,8 bis 12,8 aufweist, wobei die Alkylethercarbonsäure oder ein Salz davon eine Komponente, mit n = 0 in einer Menge von 9,8 bis 27 Masse% enthält und eine Komponente mit n = 1 und eine Komponente mit n = 2 in einer Gesamtmenge von 21 Masse% oder mehr und weniger als 40 Masse% enthält und wobei ein Mittelwert von n von 2,8 bis 3,4 beträgt, und
(B) ein Polyoxyethylenalkylsulfat, dargestellt durch Formel (2):

$$R^2\text{-O-}(CH_2CH_2O)_m\text{-}SO_3Y \qquad (2),$$

wobei $R^2$ eine Alkylgruppe oder eine Alkenylgruppe mit 8 bis 22 Kohlenstoffatomen darstellt, m eine Zahl von 0 bis 20 darstellt, ein Mittelwert von m 2 oder mehr und weniger als 4 beträgt und Y ein Wasserstoffatom oder ein Kation, ausgewählt aus Alkalimetall, Erdalkalimetall, Ammonium, Alkylammonium, Alkanolammonium und Glucammonium, darstellt.

2. Hautreinigungszusammensetzung gemäß Anspruch 1, wobei in der Formel (1) in der Komponente (A) ein Mittelwert von n von 1,5 bis 10 beträgt.

3. Hautreinigungszusammensetzung gemäß einem der Ansprüche 1 bis 2, wobei in der Formel (1) in der Komponente (A) $R^1$ eine Alkylgruppe mit 10 bis 16 Kohlenstoffatomen darstellt, eine durchschnittliche Kohlenstoffanzahl von $R^1$ von 10,8 bis 12,5 beträgt, der Mittlwert n von 2,8 bis 3,4 beträgt, eine Komponente mit n = 0 in einer Menge von 9,9 bis 16 Masse% enthalten ist und eine Komponente mit n = 1 und eine Komponente mit n = 2 in einer Gesamtmenge von 27 bis 36,5 Masse% enthalten sind.

4. Hautreinigungszusammensetzung gemäß einem der Ansprüche 1 bis 3, wobei in der Formel (1) in der Komponente (A) (die Masse einer Komponente mit n = 0) : (die Masse einer Komponente mit n = 1) : (die Masse einer Komponente mit n = 2) : (die Masse einer Komponente mit n = 3) : (die Masse einer Komponente mit n = 4) = 1 : 0,99 bis 3,50 : 0,89 bis 3,00 : 0,76 bis 3,00 : 0,63 bis 1,52.

5. Hautreinigungszusammensetzung gemäß einem der Ansprüche 1 bis 4, wobei in der Formel (1) in der Komponente (A) $R^1$ zwei oder mehr Alkylgruppen enthält und der Gehalt einer Komponente mit einer Alkylkettenlänge, die im höchsten Gehalt enthalten ist, 55 Masse% oder mehr und weniger als 97 Masse% beträgt.

6. Hautreinigungszusammensetzung gemäß einem der Ansprüche 1 bis 5, welche eine Komponente mit n = 0 in einer Menge von 9,9 Masse% oder mehr und weniger als 12 Masse% umfasst und ein Verhältnis von (der Masse einer Komponente mit n = 0) : (der Masse einer Komponente mit n = 1) : (der Masse einer Komponente mit n = 2) : (der Masse einer Komponente mit n = 3) : (der Masse einer Komponente mit n = 4) von 1 : 1,53 bis 1,87 : 1,59 bis 2,25 : 1,33 bis 2,16 : 1,14 bis 1,52 aufweist oder eine Komponente mit n = 0 in einer Menge von 1,2 Masse% oder mehr und 17 Masse% oder weniger umfasst und ein Verhältnis von (der Masse einer Komponente mit n = 0) : (der Masse einer Komponente mit n = 1) : (der Masse einer Komponente mit n = 2) : (der Masse einer Komponente mit n = 3) : (der Masse einer Komponente mit n = 4) von 1 : 0,99 bis 1,34 : 0,89 bis 1,40 : 0,76 bis 1,23 : 0,63 bis 0,99 in der Formel (1)in der Komponente (A) aufweist.

7. Hautreinigungszusammensetzung gemäß einem der Ansprüche 1 bis 6, wobei ein Massenverhältnis der Komponente (A) zu der Komponente (B), (A) : (B), von 1 : 10 bis 10 : 1 beträgt.

8. Hautreinigungszusammensetzung gemäß einem der Ansprüche 1 bis 7, wobei ein Massenverhältnis der Komponente (A) zu der Komponente (B), (A) : (B), von 1 : 5 bis 5 : 1 beträgt.

9. Hautreinigungszusammensetzung gemäß einem der Ansprüche 1 bis 8, wobei ein Massenverhältnis der Komponente (A) zu der Komponente (B), (A) : (B), von 1 : 2 bis 2 : 1 beträgt.

10. Hautreinigungszusammensetzung gemäß einem der Ansprüche 1 bis 9, ferner umfassend (C) ein nicht-ionisches Tensid.

11. Verfahren zur Hautreinigung, umfassend Auftragen der Hautreinigungszusammensetzung gemäß einem der Ansprüche 1 bis 10 auf einen Hautbereich, Waschen und dann Abspülen.

12. Verwendung der Reinigungszusammensetzung gemäß einem der Ansprüche 1 bis 11 als Hautreinigungsmittel.

**Revendications**

1. Composition nettoyante pour la peau, comprenant les composants (A) et (B) suivants :

(A) un acide carboxylique d'éther d'alkyle ou l'un de ses sels représenté par la formule (1)

$$R^1\text{-O-}(CH_2CH_2O)_n\text{-}CH_2\text{-COOM} \qquad (1)$$

dans laquelle, $R^1$ représente un groupe alkyle comportant de 10 à 16 atomes de carbone, n représente un nombre de 0 à 20, et M représente un atome d'hydrogène, un métal alcalin, un métal alcalinoterreux, un ammonium, ou un ammonium organique,
dans laquelle, $R^1$ présente un nombre moyen d'atomes de carbone de 10,8 à 12,8,
dans laquelle, l'acide carboxylique d'éther d'alkyle ou l'un de ses sels contient un composant dans lequel n = 0 dans une quantité de 9,8 à 27 % en masse, et un composant dans lequel n = 1 et un composant dans lequel n = 2 dans une quantité totale de 21 % en masse ou supérieure et inférieure à 40 % en masse, et la valeur moyenne de n est de 2,8 à 3,4, et
(B) un sulfate d'alkyle polyoxyéthyléné représenté par la formule (2) :

$$R^2\text{-O-}(CH_2CH_2O)_m\text{-}SO_3Y \qquad (2)$$

dans laquelle, $R^2$ représente un groupe alkyle ou un groupe alcényle comportant de 8 à 22 atomes de carbone, m représente un nombre de 0 à 20 et la moyenne de m est de 2 ou supérieure et inférieure à 4, et Y représente un atome d'hydrogène ou un cation choisi parmi un métal alcalin, un métal alcalinoterreux, un ammonium, un alkyl-ammonium, un alcanol-ammonium, et un glucammonium.

2. Composition nettoyante pour la peau selon la revendication 1, dans laquelle, dans le composant (A), dans la formule (1), la valeur moyenne de n est de 1,5 à 10.

3. Composition nettoyante pour la peau selon l'une quelconque des revendications 1 à 2, dans laquelle, dans le composant (A), dans la formule (1), $R^1$ représente un groupe alkyle comportant de 10 à 16 atomes de carbone, le nombre moyen d'atomes de carbone de $R^1$ est de 10,8 à 12,5, la valeur moyenne de n est de 2,8 à 3,4, un composant dans lequel n = 0 est contenu dans une quantité de 9,9 à 16 % en masse, et un composant dans lequel n = 1 et un composant dans lequel n = 2 sont contenus dans une quantité totale de 27 à 36,5 % en masse.

4. Composition nettoyante pour la peau selon l'une quelconque des revendications 1 à 3, dans laquelle, dans le composant (A), dans la formule (1), (la masse d'un composant dans lequel n = 0) / (la masse d'un composant dans lequel n = 1) / (la masse d'un composant dans lequel n = 2) / (la masse d'un composant dans lequel n = 3) / (la masse d'un composant dans lequel n = 4) = 1 / 0,99 à 3,50 / 0,89 à 3,00 / 0,76 à 3,00 / 0, 63 à 1,52.

5. Composition nettoyante pour la peau selon l'une quelconque des revendications 1 à 4, dans laquelle, dans le composant (A), dans la formule (1), $R^1$ contient deux groupes alkyle ou plus, et la teneur en un composé comportant une longueur de chaîne alkyle qui est contenue dans la teneur la plus élevée est de 55 % en masse ou supérieure et inférieure à 97 % en masse.

6. Composition nettoyante pour la peau selon l'une quelconque des revendications 1 à 5, qui comprend un composant dans lequel n = 0 dans une quantité de 9,9 % en masse ou supérieure et inférieure à 12 % en masse et présente un rapport de (la masse d'un composant dans lequel n = 0) / (la masse d'un composant dans lequel n = 1) / (la masse d'un composant dans lequel n = 2) / (la masse d'un composant dans lequel n = 3) / (la masse d'un composant dans lequel n = 4) de 1 / 1, 53 à 1, 87 / 1,59 à 2,25 / 1,33 à 2,16 / 1,14 à 1,52, ou comprend un composant dans lequel n = 0 dans une quantité de 12 % ou supérieure et de 17 % en masse ou inférieure et présente un rapport de (la masse d'un composant dans lequel n = 0) / (la masse d'un composant dans lequel n = 1) / (la masse d'un composant dans lequel n = 2) / (la masse d'un composant dans lequel n = 3) / (la masse d'un composant dans lequel n = 4) de 1 / 0,99 à 1,34 / 0,89 à 1,40 / 0,76 à 1,23 / 0,63 à 0,99, dans le composant (A), dans la formule (1).

7. Composition nettoyante pour la peau selon l'une quelconque des revendications 1 à 6, dans laquelle le rapport massique du composant (A) sur le composant (B), (A) / (B), est de 1 / 10 à 10 / 1.

8. Composition nettoyante pour la peau selon l'une quelconque des revendications 1 à 7, dans laquelle le rapport massique du composant (A) sur le composant (B), (A) / (B), est de 1 / 5 à 5 / 1.

9. Composition nettoyante pour la peau selon l'une quelconque des revendications 1 à 8, dans laquelle le rapport massique du composant (A) sur le composant (B), (A) / (B), est de 1 / 2 à 2 / 1.

10. Composition nettoyante pour la peau selon l'une quelconque des revendications 1 à 9, comprenant en outre (C) un tensioactif non ionique.

11. Procédé de nettoyage de la peau, comprenant une application de la composition nettoyante pour la peau selon l'une quelconque des revendications 1 à 10 sur une zone de la peau, un lavage, et ensuite un rinçage.

12. Utilisation de la composition nettoyante selon l'une quelconque des revendications 1 à 11 en tant qu'agent nettoyant pour la peau.

**EP 2 752 186 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 960579 A
- JP H02175799 A
- JP 2009263290 A
- JP 2008285479 A